# EUROPEAN PATENT SPECIFICATION

(11) **EP 1 755 568 B1**
(45) Date of publication and mention of the grant of the patent: **12.08.2015**
(21) Application number: 05733390.8
(22) Date of filing: 31.03.2005
(51) Int. Cl.: A61K 9/52, A61K 31/155, A61K 9/20, A61K 47/10, A61K 47/32, A61K 47/36

(54) **CONTROLLED RELEASE FORMULATION FOR ORAL ADMINISTRATION OF METFORMIN**
FORMULIERUNG MIT KONTROLLIERTER FREISETZUNG ZUR ORALEN VERABREICHUNG VON METFORMIN
FORMULATION A LIBERATION LENTE POUR L'ADMINISTRATION ORALE DE METFORMINE

(30) Priority: 01.04.2004 KR 2004022527
(43) Date of publication of application: 28.02.2007
(73) Proprietor: Hanmi Pharm. Co., Ltd., Hwaseong-gun, Kyungki-do 445-910 (KR)
(72) Inventor: WOO, Jong Soo Daewol Maeul 821-105;, Suwon-si, Kyungki-do 440-300 (KR); KIM, Young Hun, Jangan -gu, Suwon-si Kyungki-do 440-320 (KR)
(74) Representative: Grünecker Patent- und Rechtsanwälte PartG mbB
(86) International application number: PCT/KR2005/000936
(87) International publication number: WO 2005/094794

(56) References cited:
- EP-A1- 0 998 271
- WO-A1-03/004009
- WO-A1-2004/110422
- WO-A1-2005/060942
- US-A- 5 955 106
- US-A1- 2004 052 848
- US-A1- 2004 109 891
- US-B1- 6 517 866
- US-B2- 6 340 475
- APICELLA A ET AL: "POLY(ETHYLENE OXIDE) (PEO) AND DIFFERENT MOLECULAR WEIGHT PEO BLENDS MONOLITHIC DEVICES FOR DRUG RELEASE" BIOMATERIALS, ELSEVIER SCIENCE PUBLISHERS BV., BARKING, GB, vol. 14, no. 2, 1 January 1993 (1993-01-01), pages 83-90, XP000335514 ISSN: 0142-9612
- LALOR B.C. ET AL: 'Placebo-controlled trial of the effects of sugar gum and metformin on fasting blood glucose and serum lipids in obese, type 2 diabetic patients' UNIVERSITY OF MANCHESTER DEPARTMENT OF MEDICINE, DIABETIC MEDICINE vol. 7, no. 3, 1990, pages 242 - 245, XP008125281

## Description

### Field of the Invention

The present invention relates to a controlled release formulation for oral administration of metformin or a pharmaceutically acceptable salt thereof.

### Background of the Invention

Metformin is an oral medication designed to help control elevated blood sugar levels in non-insulin dependent diabetes mellitus (NIDDM) by activating glucose receptor in liver. It induces weight loss, reduces blood-triglyceride level and low-density lipoproteins (LDL), and increases high-density lipoproteins (HDL) in diabetic patient. Therefore, it may be used as a primary drug for NIDDM.

Metformin is currently marketed in the form of a hydrochloride as GLUCOPHAGE^{®} (Bristol-myers Squibb Company) tablets and its daily dosage is determined individually on the basis of both effectiveness and tolerance, while not exceeding the maximum recommended dose of 2,550 mg per day. The side effects of metformin are loss of appetite, abdominal distension, nausea and diarrhea while skin eruption or hives may break out rarely. These side effects may be avoided by reducing the minimum and/or maintenance dose, or by administrating a controlled release formulation.

Existing controlled release formulations of metformin are based on the use of polymers or release control by osmotic pressure. For example, WO 99/47128 discloses a two phase controlled release system based on polymers such as ethyl cellulose, sodium carboxy methyl cellulose and hydroxy propyl methyl cellulose with high water soluble medicament; WO 02/36100 discloses a method for controlling the release of medicament through the use of a perforated controlled release coating; and United States Patent No. 3,952,741 teaches an osmotic device comprising a semi permeable membrane.

However, the existing controlled release formulations have the problem of high production costs and/or unsatisfactory release patterns.

Therefore, there has been a continual need to develop an economic controlled release formulation of metformin, which is capable of maintaining the effectiveness of the drug by uniform release over a prescribed period.

### Summary of the Invention

Accordingly, it is an object of the present invention to provide a controlled release formulation of metformin, which can maintain uniform release of metformin for a long period of time and can be prepared easily.

In accordance with one aspect of the present invention, there is provided a controlled release formulation for oral administration of metformin or a pharmaceutically acceptable salt thereof comprising metformin or a pharmaceutically acceptable salt thereof as a pharmaceutically active ingredient; a combination of a polyethylene oxide and a xanthan gum as a carrier for controlled release; and a pharmaceutically acceptable additive.

### Brief Description of Drawings

The above and other objects and features of the present invention will become apparent from the following description of the invention taken in conjunction with the following accompanying drawings, which respectively show:
Fig. 1: *in vitro* release profiles of controlled release tablets prepared in Examples 1 to 4 of the present invention, and a comparative formulation (GLUCOPHAGE^{®} XR controlled release tablet, Bristol-Myers Squibb Company);
Fig. 2: *in vitro* release profiles of the controlled release tablets prepared in Examples 5 to 8 of the present invention, and a comparative formulation (GLUCOPHAGE^{®} XR controlled release tablet);
Fig. 3: *in vitro* release profiles of the controlled release tablets prepared in Examples 9 to 12 of the present invention, and a comparative formulation (GLUCOPHAGE^{®} XR controlled release tablet);
Fig. 4: *in vitro* release profiles of the controlled release tablets prepared in Example 2, and Comparative Examples 1 and 2;
Fig. 5: *in vitro* release profiles of the controlled release tablet prepared in Example 12 of the present invention as function of the rotation speed of the release port; and
Fig. 6: *in vitro* release profiles of a comparative formulation (GLUCOPHAGE^{®} XR controlled release tablet) as function of the rotation speed of the release port.

### Detailed Description of the Invention

The controlled release formulation for oral administration of metformin may be achieved by mixing a suitable metformin salt with a hydrophilic polymer to form solid particles. Employing a homologous and/or a heterologous hydrophilic polymer, the particles may be dispersed to formulate a compressed tablet or a packed capsule.

Each ingredient of said formulation is described in detail as follows:

### (1) Pharmaceutically active ingredient

The active ingredient of the controlled release formulation of the present invention is metformin or its pharmaceutically acceptable salt, e.g., a hydrochloride, succinate or fumarate.

### (2) Carrier for controlled release

The carrier for controlled release of the present invention is a combination of a polyethylene oxide and a natural gum. The polyethylene oxide may be chosen from the ones having average molecular weight between 100,000 and 7,000,000, or a mixture of two or more polyethylene oxides with different molecular weights may be also used.

The natural gum of the present invention refers to xanthan gum.

In accordance with the present invention, the weight ratio of the active ingredient : the carrier for controlled release may range from 1 : 0.01 to 1 : 1, and preferably, from 1 : 0.1 to 1 : 0.95.

### (3) Pharmaceutically acceptable additive

The ingredients that can be supplemented to the formulation for controlled release include pharmaceutical additives acceptable for a solid formulation for oral administration such as neutralized diluent carriers, binders and lubricants.

The neutralized diluent carrier of the present invention can be lactose, dextrin, starch, microcrystallized cellulose, potassium phosphate monobasic, calcium carbonate, saccharide or silicon dioxide, and the like, which may contain conventional additives in the pharmaceutical field used in solid formulations for oral administration.

The binders of the present invention can be polyvinyl pyrrolidone or gelatin. Other conventional additives in the pharmaceutical field that are applied to solid formulation for oral administration can be also included.

The lubricants of the present invention can be a zinc or magnesium salt of stearic acid and the like, which may contain conventional additives in the pharmaceutical field used in solid formulations for oral administration.

In accordance with the present invention, the weight ratio of the active ingredient: the pharmaceutically acceptable additives may range from 1 : 0.001 to 1 : 0.3, preferably, from 1 : 0.01 to 1 : 0.1.

In order to have a more delicate control of active ingredient release, a selective release-controlling agent such as a wax or a polyvinyl acetate/polyvinyl pyrrolidone mixture, which helps the carrier for controlled release in manifesting its gel property *in vivo,* may be additionally used as an optional ingredient in the formulation of the present invention.

The weight ratio of the active ingredient : the said selective release controlling agent may preferably range from 1 : 0 to 1 : 0.9, whereas the ratio of total weight of the formulation : the agent can preferably range from 1 : 0 to 1 : 0.7.

The following Examples are intended to further illustrate the present invention without limiting its scope.

### Examples: Preparation of Metformin Controlled Release Tablet

### Example 1

500g of metformin·HCl (Hwail Pharm. Co., Ltd), 80g of polyethylene oxide (Polyox^{®} WSR Agglutinant, Molecular weight 5,000,000, Union Carbide) and 100 g of xanthan gum (Cpkelco) were each filtered through No. 30 mesh and mixed together. The mixture was placed in a high-speed mixer (SPG-2, Fujipaudal), and a binder solution made up of 20g of polyvinyl pyrrolidone (Kollidon^{®} K-90, BASF) dissolved in distilled water was added to the mixer, followed by mixing at a speed of 100∼1,000 rpm for 3min to obtain granules. The granules were dried and filtered through No. 30 mesh. Thereafter, 200g of a polyvinyl acetate/polyvinyl pyrrolidone mixture (Kollidon SR, BASF), 80g of wax (Compritol^{®} 888ATO, Gattefosse) and 10g of silicon dioxide were added to the granules and mixed for 30 min. Finally, 10g of magnesium stearate powder was added to the mixture, mixed for 3 min, and compressed to obtain a tablet having the composition of Table 1.

**Table 1**

| Ingredients | | Content (wt%) |
|---|---|---|
| Granule forming part | Metformin · HCl | 50 |
| | Polyethylene oxide (Polyox^{®} WSR, M.W 5,000,000) | 8 |
| | Xanthan gum | 10 |
| | Polyvinyl pyrrolidone | 2 |
| Mixture part | Polyvinyl acetate/Polyvinyl pyrrolidone mixture | 20 |
| | Wax | 8 |
| | Silicon dioxide | 1 |
| | Magnesium stearate | 1 |
| Total | | 100 |

### Examples 2 to 5

Tablets having the compositions listed in Tables 2 to 5 were prepared by repeating the procedure of Example 1 except for using Xanthan gum (Cpkelco) in the mixture part or using polyethylene oxides having different molecular weights. In addition, the binder, polyvinyl pyrrolidone was also excluded from the granule forming part in these examples.

**Table 2: Composition of a tablet of Example 2**

| Ingredients | | Content (wt%) |
|---|---|---|
| Granule forming part | Metformin · HCl | 50 |
| | Polyethylene oxide (Polyox^{®} WSR, M.W 5,000,000) | 5 |
| Mixture part | Polyvinyl acetate/Polyvinyl pyrrolidone mixture | 20 |
| | Wax | 13 |
| | Xanthan gum | 10 |
| | Silicon dioxide | 1 |
| | Magnesium stearate | 1 |
| Total | | 100 |

**Table 3: Composition of a tablet of Example 3**

| Ingredients | | Content (wt%) |
|---|---|---|
| Granule forming part | Metformin · HCl | 50 |
| | Polyethylene oxide (Polyox^{®} WSR N10, M.W 100,000) | 5 |
| Mixture part | Polyvinyl acetate/Polyvinyl pyrrolidone mixture | 20 |
| | Wax | 13 |
| | Xanthan gum | 10 |
| | Silicon dioxide | 1 |
| | Magnesium stearate | 1 |
| Total | | 100 |

**Table 4: Composition of a tablet of Example 4**

| Ingredients | | Content (wt%) |
|---|---|---|
| Granule forming part | Metformin · HCl | 50 |
| | Polyethylene oxide (Polyox^{®} WSR 1105, M.W 900,000) | 5 |
| Mixture part | Polyvinyl acetate/Polyvinyl pyrrolidone mixture | 20 |
| | Wax | 13 |
| | Xanthan gum | 10 |
| | Silicon dioxide | 1 |
| | Magnesium stearate | 1 |
| Total | | 100 |

**Table 5: Composition of a tablet of Example 5**

| Ingredients | | Content (wt%) |
|---|---|---|
| Granule forming part | Metformin · HCl | 50 |
| | Polyethylene oxide (Polyox^{®} WSR, M.W 5,000,000) | 10 |
| Mixture part | Polyvinyl acetate/Polyvinyl pyrrolidone mixture | 20 |
| | Wax | 8 |
| | Xanthan gum | 10 |
| | Silicon dioxide | 1 |
| | Magnesium stearate | 1 |
| Total | | 100 |

### Example 6

A tablet having the composition shown in Table 6 was prepared by repeating the procedure of Example 1 except for not using the binder, polyvinyl pyrrolidone.

**Table 6**

| Ingredients | | Content (wt%) |
|---|---|---|
| Granule forming part | Metformin · HCl | 50 |
| | Polyethylene oxide (Polyox^{®} WSR, M.W 5,000,000) | 10 |
| | Xanthan gum | 10 |
| Mixture part | Polyvinyl acetate/Polyvinyl pyrrolidone mixture | 20 |
| | Wax | 8 |
| | Silicon dioxide | 1 |
| | Magnesium stearate | 1 |
| Total | | 100 |

### Example 7

A tablet having the composition shown in Table 7 was prepared by repeating the procedure of Example 1 except for using isopropyl alcohol in place of distilled water during the granule formation step.

**Table 7**

| Ingredients | | Content (wt%) |
|---|---|---|
| Granule forming part | Metformin · HCl | 50 |
| | Polyethylene oxide (Polyox^{®} WSR, M.W 5,000,000) | 8 |
| | Xanthan gum | 10 |
| | Polyvinyl pyrrolidone | 2 |
| Mixture part | Polyvinyl acetate/Polyvinyl pyrrolidone mixture | 20 |
| | Wax | 8 |
| | Silicon dioxide | 1 |
| | Magnesium stearate | 1 |
| Total | | 100 |

### Examples 8 to 10

Tablets having the compositions shown in Tables 8 to 10 were prepared by repeating the procedure of Example 1 except for using a distilled water/isopropyl alcohol mixture (1:1 (v/v)) in place of distilled water during the granule formation step and not using the wax.

**Table 8: Composition of a tablet of Example 8**

| Ingredients | | Content (wt%) |
|---|---|---|
| Granule forming part | Metformin · HCl | 50 |
| | Polyethylene oxide (Polyox^{®} WSR, M.W 5,000,000) | 8 |
| | Xanthan gum | 10 |
| | Polyvinyl pyrrolidone | 2 |
| Mixture part | Polyvinyl acetate/Polyvinyl pyrrolidone mixture | 28 |
| | Silicon dioxide | 1 |
| | Magnesium stearate | 1 |
| Total | | 100 |

**Table 9: Composition of a tablet of Example 9**

| Ingredients | | Content (wt%) |
|---|---|---|
| Granule forming part | Metformin · HCl | 50 |
| | Polyethylene oxide (Polyox^{®} WSR, M.W 5,000,000) | 16 |
| | Xanthan gum | 10 |
| | Polyvinyl pyrrolidone | 2 |
| Mixture part | Polyvinyl acetate/Polyvinyl pyrrolidone mixture | 20 |
| | Silicon dioxide | 1 |
| | Magnesium stearate | 1 |
| Total | | 100 |

**Table 10: Composition of a tablet of Example 10**

| Ingredients | | Content (wt%) |
|---|---|---|
| Granule forming part | Metformin · HCl | 50 |
| | Polyethylene oxide (Polyox^{®} WSR, M.W 5,000,000) | 8 |
| | Xanthan gum | 18 |
| | Polyvinyl pyrrolidone | 2 |
| Mixture part | Polyvinyl acetate/Polyvinyl pyrrolidone mixture | 20 |
| | Silicon dioxide | 1 |
| | Magnesium stearate | 1 |
| Total | | 100 |

### Example 11

A tablet having the composition shown in Table 11 was prepared by repeating the procedure of Example 1 except for using a distilled water/isopropyl alcohol mixture (1:1 (v/v)) during the granule formation step as well as using xanthan gum (Cpkelco) and locust bean gum (Sigma) in the mixture part while not using the wax.

**Table 11**

| Ingredients | | Content (wt%) |
|---|---|---|
| Granule forming part | Metformin · HCl | 50 |
| | Polyethylene oxide (Polyox^{®} WSR, M.W 5,000,000) | 10 |
| | Polyvinyl pyrrolidone | 2 |
| Mixture part | Polyvinyl acetate/Polyvinyl pyrrolidone mixture | 20 |
| | Xanthan gum | 10 |
| | Locust bean gum | 6 |
| | Silicon dioxide | 1 |
| | Magnesium stearate | 1 |
| Total | | 100 |

### Example 12

A tablet having the composition listed in Table 12 was prepared by repeating the procedure of Example 11 except for not using the polyvinyl acetate/polyvinyl pyrrolidone mixture.

**Table 12**

| Ingredients | | Content (wt%) |
|---|---|---|
| Granule forming part | Metformin · HCl | 50 |
| | Polyethylene oxide (Polyox^{®} WSR, M.W 5,000,000) | 10 |
| | Polyvinyl pyrrolidone | 2 |
| Mixture part | Xanthan gum | 21 |
| | Locust bean gum | 15 |
| | Silicon dioxide | 1 |
| | Magnesium stearate | 1 |
| Total | | 100 |

### Comparative Example 1

The tablet having the composition listed in Table 13 was prepared by repeating the procedure of Example 2 except for not using polyethylene oxide during granule formation.

**Table 13**

| Ingredients | | Content (wt%) |
|---|---|---|
| Granule forming part | Metformin · HCl | 52.6 |
| Mixture part | Polyvinyl acetate/Polyvinyl pyrrolidone mixture | 21.1 |
| | Wax | 13.7 |
| | Xanthan gum | 10.5 |
| | Silicon dioxide | 1.1 |
| | Magnesium stearate | 1 |
| Total | | 100 |

### Comparative example 2

A tablet having the composition listed in Table 14 was prepared by repeating the procedure of Example 2 except for not using xanthan gum.

**Table 14**

| Ingredients | | Content (wt%) |
|---|---|---|
| Granule forming part | Metformin · HCl | 55.6 |
| | Polyethylene oxide (Polyox^{®} WSR, M.W 5,000,000) | 5.6 |
| Mixture part | Polyvinyl acetate/Polyvinyl pyrrolidone mixture | 22.2 |
| | Wax | 14.4 |
| | Silicon dioxide | 1.1 |
| | Magnesium stearate | 1.1 |
| Total | | 100 |

### Test Example 1: In vitro Release-Test

The tablets prepared in Examples 1 to 12 and GLUCOPHAGE^{®} XR controlled release tablet (Bristol-Myers Squibb Company) as a comparative formulation were subjected to *in vitro* release-test in accordance with the release-test method described in Korea pharmacopoeia (the paddle method) to compare the effects of natural gum and polyethylene oxide as carriers for controlled release on the release speed. The release patterns of metformin·HCl from each of the tablets were measured under the following conditions.
- Release-test system: Erweka DT 80
- Release solution: The disintegrating-test 2nd method described in Korea pharmacopoeia (artificial gastric fluid)
- Temperature of release solution: 37 ± 0.5 °C
- Amount of release solution: 900 mL
- Rotation speed: 50 rpm
- Sample collection time: Aliquots of the release solution were collected at 1, 2, 3, 4, 6, 8, and 10 hr, filtered through a 0.45 µm membrane, and used as test samples. After sampling the release solution, the release-test system was refilled with an equal amount of fresh release solution.
- Analyzing method: Absorbances of the samples and a standard solution were measured at 233nm employing distilled water as a reference to calculate corresponding release ratios.
- Calculation of released amount: Cumulative release amount

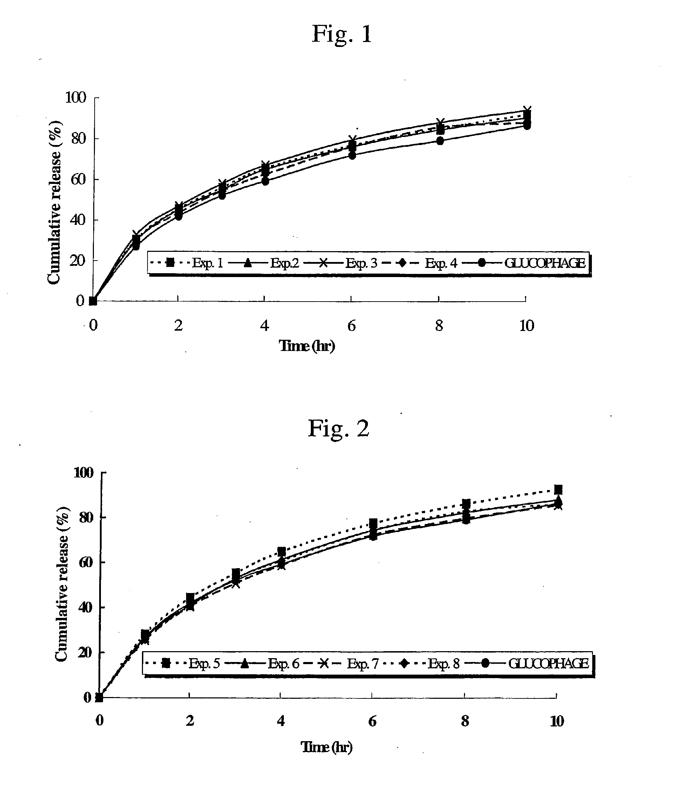

As can be seen from Figs. 1 to 3, the release rate becomes slow as the amount of polyethylene oxide or the natural gum increases. Especially, the tablet of Example 14 releases the drug continuously in a release pattern similar to that of the comparative formulation.

### Test Example 2: In vitro Release-Test

*In vitro* release-tests were conducted by repeating the method of Test Example 1, except for using the tablets prepared in Example 2, and Comparative Examples 1 and 2.

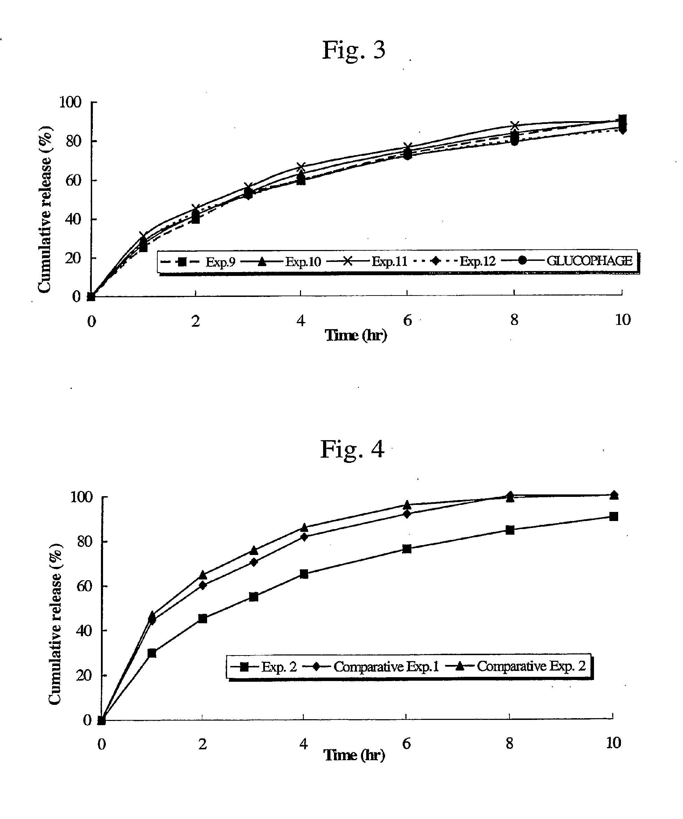

As can be seen from Fig. 4, tablets of Comparative Examples 1 and 2, which contain natural gum or polyethylene oxide alone as a carrier for controlled release show burst drug releases at the initial stage.

### Test Example 3: In vitro Release-Test

*In vitro* release-tests were conducted for the tablet prepared in Example 12 and the comparative formulation by repeating the method of Test Example 1, except for changing the rotation speed to 100 rpm and 150 rpm.

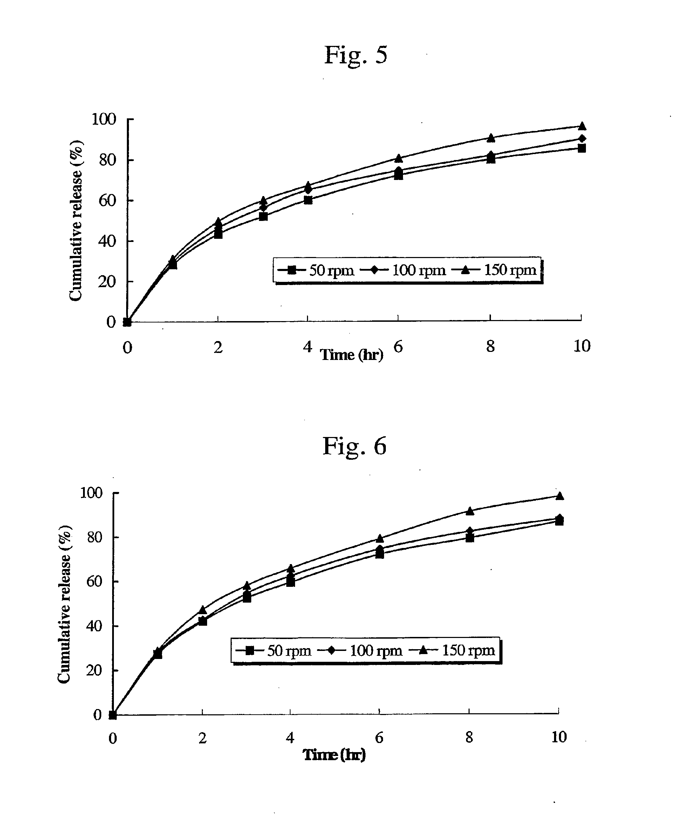

As can be seen from Figs. 5 and 6, the tablet of Example 12 displays a steady release pattern equal to that of the comparative formulation, without initial burst release of the drug even at a high rotation speed.

While the invention has been described with respect to the above specific embodiments, it should be recognized that various modifications and changes may be made and also fall within the scope of the invention as defined by the claims that follow.

## Claims

1. A controlled release formulation for oral administration of metformin or a pharmaceutically acceptable salt thereof comprising metformin or a pharmaceutically acceptable salt thereof as an active ingredient; a combination of a polyethylene oxide and xanthan gum as a carrier for controlled release; and a pharmaceutically acceptable additive, wherein the weight ratio of metformin or a pharmaceutically acceptable salt thereof: carrier ranges from 1:0.01 to 1:1.

2. The controlled release formulation of claim 1, wherein the pharmaceutically acceptable salt of metformin is metformin hydrochloride, metformin succinate or metformin fumarate.

3. The controlled release formulation of claim 1, wherein the polyethylene oxide has an average molecular weight in the range of 100,000 to 7, 000, 000.

## Patentansprüche

1. Eine Formulierung mit kontrollierter Freisetzung zur oralen Verabreichung von Metformin oder eines pharmazeutisch verträglichen Salzes davon, umfassend Metformin oder ein pharmazeutisch verträgliches Salz davon als Wirkstoff; eine Kombination aus einem Polyethylenoxid und Xanthangummi als Träger für die kontrollierte Freisetzung; und einen pharmazeutisch akzeptablen Zusatzstoff, wobei das Gewichtsverhältnis von Metformin oder einem pharmazeutisch verträglichen Salz davon: Träger im Bereich von 1: 0,01 bis 1: 1 liegt.

2. Formulierung mit kontrollierter Freisetzung nach Anspruch 1, wobei das pharmazeutisch verträgliche Salz von Metformin Metformin-Hydrochlorid, Metformin Succinat oder Metformin Fumarat ist.

3. Formulierung mit kontrollierter Freisetzung nach Anspruch 1, wobei das Polyethylenoxid ein durchschnittliches Molekulargewicht im Bereich von 100.000 bis 7.000.000 aufweist.

## Revendications

1. Formulation à libération contrôlée pour l'administration orale de metformine ou d'un sel pharmaceutiquement acceptable de celle-ci, comprenant de la metformine ou un sel pharmaceutiquement acceptable de celle-ci comme principe actif ; une combinaison d'oxyde de polyéthylène et de gomme xanthane comme porteur de libération contrôlée ; et un additif pharmaceutiquement acceptable, dans laquelle le rapport massique de la metformine ou du sel pharmaceutiquement acceptable de celle-ci au porteur est compris entre 1:0,01 et 1:1.

2. Formulation à libération contrôlée selon la revendication 1, dans laquelle le sel de metformine pharmaceutiquement acceptable est l'hydrochlorure de metformine, le succinate de metformine ou le fumarate de metformine.

3. Formulation à libération contrôlée selon la revendication 1, dans laquelle l'oxyde de polyéthylène présente une masse moléculaire moyenne comprise entre 100.000 et 7.000.000.
